# EUROPEAN PATENT APPLICATION

(11) **EP 4 548 930 A1**
(43) Date of publication of application: **07.05.2025**
(21) Application number: 23830395.2
(22) Date of filing: 29.06.2023
(51) Int. Cl.: A61K 39/395, A61K 39/00, C07K 16/28, C07K 16/30, A61P 35/00

(54) **PHARMACEUTICAL COMPOSITION COMPRISING ANTI-CTLA4 AND ANTI-PD1 ANTIBODY MIXTURE AND THERAPEUTIC USE THEREOF**

(30) Priority: 30.06.2022 CN 202210763564; 09.01.2023 CN 202310037793
(71) Applicant: QILU PHARMACEUTICAL CO., LTD., Jinan, Shandong 250100 (CN)
(72) Inventor: WANG, Xiaofei, Jinan, Shandong 250100 (CN); LI, Lingyan, Jinan, Shandong 250100 (CN); YU, Xiaokui, Jinan, Shandong 250100 (CN); XUE, Shilin, Jinan, Shandong 250100 (CN); WANG, Yimei, Jinan, Shandong 250100 (CN)
(74) Representative: Bandpay & Greuter
(86) International application number: PCT/CN2023/103768
(87) International publication number: WO 2024/002226

(57) **Abstract**

The present disclosure relates to a mixture of antibodies of anti-CTLA4 and anti-PD1 or use of the mixture of antibodies in combination with a chemotherapeutic drug and/or an anti-angiogenic agent to treat hepatocellular carcinoma.

## Description

### Technical Field

The present disclosure relates to the treatment of cancer, particularly hepatocellular carcinoma, including immunotherapy and combination therapy. More specifically, the present disclosure relates to the use of a pharmaceutical composition comprising a mixture of antibodies of anti-CTLA4 and anti-PD1 for treating hepatocellular carcinoma.

### Technical Background

In China, primary liver cancer is the fifth common malignant tumor and the second leading cause of tumor death, with about 400 thousand new cases with liver cancer and 391 thousand deaths, accounting for 44% and 47% of the world's total, respectively, seriously threatening the lives and health of the people. Hepatocellular carcinoma (HCC) is the main pathological type of primary liver cancer, accounting for 85%~90%. The field of HCC treatment is characterized by multidisciplinary participation, coexistence of multiple treatment methods. Common treatment methods include hepatectomy, liver transplantation, ablation treatment, transcatheter arterial chemoembolization, radiotherapy, systemic anti-tumor treatment, and the like. Efficacy can be maximized by selecting reasonable therapies for patients with liver cancer at different stages. Due to the hidden onset of HCC, less than 30% of patients at the time of the first diagnosis are eligible for radical treatment, whereas more than two thirds of patients with HCC are already at an advanced stage at the time of diagnosis and are therefore not eligible for curative therapy.

Surgical resection, local ablation, and liver transplantation are potential curative means, but tumor recurrence is very common. The 5-year tumor recurrence rate after surgical resection or local ablation treatment is as high as 70%, and currently there is no globally recognized postoperative adjuvant treatment regimen for liver cancer after radical treatment of HCC. For patients with high-risk recurrence factors, significant clinical attention is given, and interventional measures are often actively taken, with a hope that recurrence can be prevented or delayed, which includes antiviral medications, hepatic artery interventional therapies, oxaliplatin-containing systemic chemotherapies, molecular targeted therapeutic drugs, and traditional Chinese medicines. These therapies may have certain therapeutic effects, but except for antiviral medications, there is a lack of strong evidences from evidence-based medicine to adequately support other therapies. Therefore, there is a significant unmet clinical need to develop adjuvant treatment regimens after radical treatment to prevent tumor recurrence.

For unresectable or metastatic HCC, systemic anti-tumor therapy can control disease progression and prolong patient survival, including treatment with molecular targeted drugs, immunotherapy, chemotherapy, and traditional Chinese medicine, among others. Molecular targeted drugs, such as sorafenib, have a relatively limited effective rate. HCC has poor sensitivity to chemotherapies such as those based primarily on oxaliplatin. Chemotherapy alone has limited effectiveness. First-line therapeutic drug development is at a bottleneck stage over a long period of time. With progresses in immunological research, immune checkpoint inhibitors (ICIs) have the advantages of killing residual cancer cells, improving sensitivity to chemotherapy, alleviating side effects, relieving late symptoms, and prolonging survival, and have produced significant and sustained therapeutic effects in many tumors including HCC. Although existing combinations of ICIs and anti-angiogenic drugs have made a significant breakthrough in the treatment of advanced HCC, when the existing combination therapies are adopted, the 5-year survival rate of patients with advanced HCC is still very low. There is still an unmet clinical need. It is necessary to further explore a new combination treatment regimen to further improve the prognosis of patients with advanced HCC.

ZPML265 is a pharmaceutical formulation of a mixture of antibodies composed of a recombinant humanized IgG1 monoclonal antibody targeting human CTLA4 and a recombinant humanized IgG4 monoclonal antibody targeting human PD1, the two different antibodies being produced by a single host cell. The mixture of antibodies specifically binds to CTLA4 and PD1 at the same time, thereby blocking two immune checkpoint signaling pathways relating to CTLA4 and B7-1/B7-2 as well as PD1 and PDL1, relieving the inhibitory effects of the two pathways on T lymphocytes, restoring their functional activity and anti-tumor immune response, which in turn achieve the goal of combatting and killing tumor in the body.

ZPML264 injection is a pharmaceutical preparation comprising only the anti-PD1 antibody in ZPML265, and the sequence of the anti-PD1 antibody is identical to the sequence of the anti-PD1 antibody component in the mixture of antibodies ZPML265. Results from previous clinical trials of ZPML264 have shown its good safety and tolerability, with no safety signals of specific uncontrollable adverse events compared to similar drugs such as marketed drugs pembrolizumab, nivolumab, toripalimab, sintilimab, and camrelizumab, and have demonstrated efficacy in multiple tumor species.

Bevacizumab is an anti-angiogenic drug that can cause abnormal vascular structures and functions to tend to a normal state. Normalization of blood vessels can increase the infiltration of immune cells to tumors, making the tumor microenvironment have more CD8 + T cells and B lymphocytes, thereby improving the tumor immune microenvironment.

Therefore, there is an unmet clinical need in patients with unresectable or metastatic hepatocellular carcinoma that have not been subjected to a systemic therapy. There is an urgent need to develop more effective medicines.

### Summary

The technical problem to be solved by the present disclosure is to provide a novel immunotherapy that is superior to the best first-line standard treatment for hepatocellular carcinoma currently approved and recognized in China, or to fill a blank in clinical relating to adjuvant therapy after surgical resection or local ablation of HCC. The present disclosure is based on a research and development that is patient' needs-centered and clinical value-oriented, which is expected to further improve the survival benefits of patients while taking safety into account, and meet the growing diversified clinical needs.

The present disclosure provides a pharmaceutical composition for treating liver cancer, particularly hepatocellular carcinoma, comprising an effective amount of a mixture of antibodies of anti-CTLA4 and anti-PD1.

Meanwhile, the present disclosure also provides a method for treating liver cancer, particularly hepatocellular carcinoma, comprising administrating a pharmaceutical composition comprising an effective amount of a mixture of antibodies of anti-CTLA4 and anti-PD1.

In some embodiments, the pharmaceutical composition further comprises a pharmaceutically acceptable carrier.

In some embodiments, the pharmaceutical composition further comprises an additional therapeutic agent, which includes, but is not limited to, a chemotherapeutic agent (a chemotherapeutic drug), a cytotoxic agent, a radiotherapeutic agent, a cancer vaccine, a cytoreductive agent, a targeted anticancer agent, an anti-angiogenic agent, a biological response modifier, a cytokine, a hormone, an anti-metastatic agent, and an immunotherapeutic agent.

In some embodiments, the additional therapeutic agent is a chemotherapeutic drug and/or an anti-angiogenic agent. A preferred chemotherapeutic drug is a systemic chemotherapy based primarily on oxaliplatin. A preferred anti-angiogenic agent is bevacizumab.

More preferably, the systemic chemotherapy based primarily on oxaliplatin is XELOX or FOLFOX4.

Preferably, the hepatocellular carcinoma is unresectable or metastatic hepatocellular carcinoma that has not been subjected to a systemic therapy, or hepatocellular carcinoma with high-risk recurrence factors after surgical resection or local ablation treatment.

In some embodiments, the mixture of antibodies produced by a single host cell comprising both a nucleic acid encoding an anti-CTLA4 antibody and a nucleic acid encoding an anti-PD1 antibody, wherein the sequences of heavy chain HCDR1, HCDR2 and HCDR3 of the anti-CTLA4 antibody are set forth in SEQ ID NOs: 1, 2 and 3, respectively; the sequences of light chain LCDR1, LCDR2 and LCDR3 of the anti-CTLA4 antibody are set forth in SEQ ID NOs:4, 5 and 6, respectively; the sequences of heavy chain HCDR1, HCDR2 and HCDR3 of the anti-PD1 antibody are set forth in SEQ ID NOs:9, 10 and 11, respectively; and the sequences of light chain LCDR1, LCDR2 and LCDR3 of the anti-PD1 antibody are set forth in SEQ ID NOs:12, 13 and 14, respectively.

In some embodiments, the sequence of heavy chain variable region of the anti-CTLA4 antibody is set forth in SEQ ID NO:7, the sequence of light chain variable region of the anti-CTLA4 antibody is set forth in SEQ ID NO:8, the sequence of heavy chain variable region of the anti-PD1 antibody is set forth in SEQ ID NO:15, and the sequence of light chain variable region of the anti-PD1 antibody is set forth in SEQ ID NO: 16.

In some embodiments, the sequence of heavy chain of the anti-CTLA4 antibody is set forth in SEQ ID NO: 17, the sequence of light chain of the anti-CTLA4 antibody is set forth in SEQ ID NO:18, the sequence of heavy chain of the anti-PD1 antibody is set forth in SEQ ID NO:19, and the sequence of light chain of the anti-PD1 antibody is set forth in SEQ ID NO:20.

The mixture of antibodies is administered at a dose of 5 mg/kg or 7.5mg/kg once every three weeks by intravenous infusion on day 1, with one treatment cycle of 21 days.

In some more specific embodiments, the mixture of antibodies is administered at a dose of 5 mg/kg or 7.5mg/kg on day 1 of each dosing cycle, with one dosing cycle lasting three weeks. Further, a chemotherapeutic drug and/or an anti-angiogenic agent may be additionally administered. Preferably, a bevacizumab injection is administered at a dose of 7.5mg/kg or 15 mg/kg on day 1 of each dosing cycle, with one dosing cycle lasting three weeks. The XELOX chemotherapy regimen is oxaliplatin in combination with capecitabine, in which oxaliplatin is administrated at a dose of 85mg/m² on day 1 of each dosing cycle, with one dosing cycle lasting three weeks; and capecitabine is administrated at a dose of 1000mg/m² from day 1 to day 14 of each cycle, with one dosing cycle lasting three weeks. The mixture of antibodies, bevacizumab and oxaliplatin are all administered by intravenous infusion. Capecitabine is administered orally.

Meanwhile, the present disclosure also provides the use of a mixture of antibodies comprising an effective amount of an anti-CTLA4 and anti-PD1 in the manufacture of a pharmaceutical composition for treating unresectable or metastatic hepatocellular carcinoma that has not been subjected to a systemic therapy. The pharmaceutical composition may additionally include a chemotherapeutic agent and/or an anti-angiogenic agent.

Further, the present disclosure also provides the use of a mixture of antibodies comprising an effective amount of an anti-CTLA4 and anti-PD1 in the manufacture of a pharmaceutical composition for treating hepatocellular carcinoma with high-risk recurrence factors after surgical resection or local ablation treatment.

Results from a phase Ib clinical study of the mixture of antibodies of the present disclosure as a monotherapy have shown that 25 patients with hepatocellular carcinoma enrolled in a 5 mg/kg dose group showed certain efficacy. Results from a phase Ib/II clinical study of the mixture of antibodies in combination with bevacizumab in the first-line treatment of advanced HCC have shown that both the 5 mg/kg dose group and the 7.5mg/kg dose group showed superior efficacy over existing standard treatments. Meanwhile, the effectiveness data of chemotherapy first-line treatment of HCC mainly based on the published chemotherapeutic regimen of ICIs in combination with oxaliplatin have shown that ICI drugs in combination with chemotherapy have a synergistic antitumor effect on advanced HCC. Thus, the combinatorial regime of the mixture of antibodies with bevacizumab and/or chemotherapy based primarily on oxaliplatin provided in the present disclosure is a novel drug development strategy that is patient' needs-centered and clinical value-oriented for the medical first-line treatment of hepatocellular carcinoma, and can largely address unmet clinical needs.

### Detailed Description

### Terms

All publications, patents, and patent applications mentioned in this specification are incorporated herein by reference to the same extent as if each individual publication, patent, or patent application had been specifically and individually indicated to be incorporated by reference.

Before the present disclosure is described in detail below, it is to be understood that the present disclosure is not limited to the specific methodologies, protocols, and reagents described herein, as these may vary. It is also to be understood that the terms used herein are for the purpose of describing particular embodiments only and are not intended to limit the scope of the present disclosure. Unless defined otherwise, all technical and scientific terms used herein have the same meaning as commonly understood by one of ordinary skill in the art to which the present disclosure pertains.

Certain embodiments disclosed herein include numerical ranges, and certain aspects of the present disclosure may be described in terms of ranges. Unless otherwise indicated, it is to be understood that the numerical ranges or the manners described in terms of ranges are for purposes of brevity and convenience only, and are not to be considered as a strict limitation on the scope of the present disclosure. Accordingly, the description in terms of ranges should be considered to specifically disclose all possible sub-ranges and all possible specific numerical points within that range, as if such sub-ranges and numerical points had been expressly written herein. The above principles apply equally regardless of the width of the numerical values. When description is made by using a range, the range includes the endpoints of the range.

In the cases relating to a measurable value, such as an amount and a temporary time duration, the term "about" refers to a change by ±20%, in certain cases by ±10%, in certain cases by ± 5%, in certain cases ± by 1%, or in certain cases ± by 0.1%, of a specified value.

As used herein, three-letter codes and single-letter codes for amino acids are described in J. Biol. Chem, 243, p3558 (1968).

As used herein, the term "antibody" typically refers to a Y-shaped tetrameric protein containing two heavy (H) polypeptide chains (HC) and two light (L) polypeptide chains (LC) held together by covalent disulfide bonds and non-covalent interactions. A natural IgG antibody has such structure. Each light chain contains a light chain variable domain (VL) and a light chain constant domain (CL). Each heavy chain contains a heavy chain variable domain (VH) and a heavy chain constant domain (CH) (also known as heavy chain constant region (CH)).

Five major classes of antibodies are known in the art: IgA, IgD, IgE, IgG and IgM, their corresponding heavy chain constant domains being referred to as α, δ, ε, γ and µ, respectively. IgG and IgA can be further divided into different subclasses. For example, IgG can be divided into IgGl, IgG2, IgG3 and IgG4; and IgA can be divided into IgAl and IgA2. A light chain of an antibody from any vertebrate species may be assigned to one of two apparently distinct types, termed κ and λ, based on the amino acid sequence of its constant domain.

The term "variable region" or "variable domain" presents significant changes in amino acid composition from one antibody to another and is primarily responsible for antigen recognition and binding. The variable regions of each light/heavy chain pair form an antigen binding site such that an intact IgG antibody has two binding sites (i.e., it is bivalent). The variable region (VH) of the heavy chain and the variable region (VL) of the light chain each contain three regions with extreme variability, referred to as hypervariable regions (HVR), or more generally, as complementarity determining regions (CDRs). VH and VL each having four backbone regions, FRs (or framework regions), denoted by FR1, FR2, FR3 and FR4, respectively. Thus, CDR and FR sequences typically occur in the following sequence of the heavy chain variable domain (VH) (or light chain variable domain (VL)): FR1-HCDR1 (LCDR1)-FR2-HCDR2 (LCDR2) -FR3-HCDR3 (LCDR3)-FR4.

Types of "antibodies" in a broad sense may include, for example, polyclonal antibodies, monoclonal antibodies, chimeric antibodies, humanized and primatized antibodies, CDR-grafted antibodies, human antibodies (including recombinantly produced human antibodies), recombinantly produced antibodies, intracellular antibodies, multispecific antibodies, bispecific antibodies, monovalent antibodies, multivalent antibodies, anti-idiotype antibodies, synthetic antibodies (including muteins and variants thereof), and the like.

The term "monoclonal antibody" refers to an antibody that is produced by a single cell clone and is substantially homogeneous, targeting only one particular epitope. Monoclonal antibody can be prepared using a variety of techniques known in the art, including hybridoma techniques, recombination techniques, phage display techniques, those with transgenic animals, synthetic techniques, or combinations of the foregoing, and the like.

It should be noted that the division of the CDRs and FRs of an antibody variable region of the present disclosure is determined according to the Kabat definition. Other naming and numbering systems, such as Chothia, IMGT or AHo, are also known to those skilled in the art. Thus, humanized antibodies comprising one or more CDRs derived from any naming system, based on the antibody sequences of the present disclosure, are expressly maintained within the scope of the present disclosure.

The term "antigen" refers to a substance that is recognized and specifically bound by an antibody or antibody binding fragment. In a broad sense, an antigen can include any immunogenic fragment or determinant of a selected target, including a single epitope, multiple epitopes, single domain, multiple domains, or intact extracellular domain (ECD) or protein.

The term "epitope" refers to a site on an antigen that specifically binds to an immunoglobulin or antibody. The epitope may be formed by adjacent amino acids, or non-adjacent amino acids juxtaposed due to tertiary folding of a protein.

The terms "polypeptide", "peptide", and "protein" are used interchangeably herein to refer to a polymer of amino acids of any length. The polymer may be linear, cyclic or branched. It may contain a modified amino acid, in particular a conservatively modified amino acid, and it may be interrupted by a non-amino acid. The terms also include, for example, an amino acid polymer that has been modified by glycosylation, lipidation, acetylation, phosphorylation, methylation, and the like.

As used herein, the term "mixture of antibodies" refers to that containing a limited number, optionally no more than two, three, four, five, six, seven, eight, nine or ten major antibody species produced from a host cell (optionally a cell from a single host cell line) that has been transfected with DNAs encoding at least two different antibodies (optionally full-length primate IgG antibodies) having different binding specificities. In some embodiments, DNAs encoding at least two different heavy chains (HC) and at least two different light chains (LC) may be introduced into the same host cell, for example, the host cell may be transfected with DNAs encoding at least two but no more than four different antibodies with different binding specificities. In some embodiments, the sequences of all transfected DNAs encoding HC and LC may be mutated, thereby altering the amino acid sequence of the antibody such that non-homologous HC/LC pairing is not facilitated, while homologous HC/LC pairing is highly facilitated. Where two different HCs are introduced into a host cell, one or both of the two different HCs may optionally be altered so that the formation of heterodimers is not facilitated. In some embodiments, only one heavy chain is altered to prevent the formation of heterodimers. In some embodiments, where DNAs encoding only two different antibodies are introduced into a host cell, only one of the antibodies encoded by the DNAs comprises one or more partner-directed alterations such that homologous HC/LC pairing is facilitated, while the other antibody does not comprise such alterations. In some embodiments, the host cell produces only two major antibody species, where each HC primarily paired with its homologous LC, and most antibodies are tetramers containing two heavy chains having the same amino acid sequence and two light chains having the same amino acid sequence (see PCT/US2017/030676).

The term "pharmaceutical composition" refers to a formulation or combination of formulations that comprises one, two or more active ingredients, allows the active ingredients contained therein to exist in a biologically effective form, and does not include additional ingredients with unacceptable toxicity to a subject to whom the formulation is administered. When the "pharmaceutical composition" is in the form of a combination of separate formulations containing two or more different active ingredients, they may be administered simultaneously, sequentially, separately or at intervals, the purpose of which is to exert biological activities of the various active ingredients, together for the treatment of the disease(s).

The term "pharmaceutical carrier" or "pharmaceutically acceptable carrier" refers to a diluent, an adjuvant (e.g., Freund's adjuvant (complete and incomplete)), an excipient, or a vehicle administered with a therapeutic agent.

The term "effective amount" refers to a dosage of a pharmaceutical formulation comprising the active ingredients of the present disclosure that, upon administration to a patient in a single dose or multiple doses, produces desired effects in the patient being treated. An effective amount can be easily determined by an attending physician as a person skilled in the art by considering a variety of factors such as race differences; weight, age, and health status; specific disease involved; severity of the disease; response of an individual patient; specific antibodies administered; administration mode; bioavailability profile of the administrated formulation; selected dosing regimen; and use of any concomitant therapy.

The terms "host cell", "host cell line" and "host cell culture" may be used interchangeably and refer to a cell with an exogenous nucleic acid introduced therein, including a progeny of such cell. Host cell includes a "transformant" and a "transformed cell", which includes an initially transformed cell and a progeny originated therefrom, regardless of the number of passages. The progeny may not be identical in nucleic acid content to the parent cell and may contain mutation(s). Included herein are mutated progenies having the same function or biological activity as those screened or selected from the initially transformed cell.

As used herein, the term "transfection" refers to the introduction of an exogenous nucleic acid into a eukaryotic cell. Transfection can be accomplished by a variety of means known in the art, including calcium phosphate-DNA co-precipitation, DEAE-dextran-mediated transfection, polybrene-mediated transfection, electroporation, microinjection, liposome fusion, lipid transfection, protoplast fusion, retroviral infection, and biolistics.

The term "isolated polynucleotide" or "isolated nucleic acid" refers to a nucleic acid molecule, DNA or RNA that has been removed from its native environment. For example, for the purposes of the present disclosure, a recombinant polynucleotide encoding a polypeptide contained in a vector is considered isolated. Other examples of an isolated polynucleotide include a recombinant polynucleotide maintained in a heterologous host cell or a (partially or substantially) purified polynucleotide in a solution. An isolated polynucleotide includes a polynucleotide molecule contained in a cell typically containing the polynucleotide molecule, but the polynucleotide molecule is present outside the chromosome or at a chromosomal location different from its native chromosomal location. An isolated RNA molecule includes an in vivo or in vitro RNA transcript of the present disclosure, as well as its positive- and negative-stranded forms and double-stranded forms. An isolated polynucleotide or nucleic acid of the present disclosure also includes such molecule produced synthetically. In addition, the polynucleotide or nucleic acid may or may not include a regulatory element such as a promoter, a ribosome binding site, or a transcription terminator.

The terms "nucleic acid molecule code", "encoding DNA sequence" and "encoding DNA" refer to the order of deoxyribonucleotides along the deoxyribonucleic acid chain. The order of these deoxyribonucleotides determines the order of the amino acids along the polypeptide (protein) chain. Thus, the nucleic acid sequence encodes an amino acid sequence.

Methods for producing and purifying an antibody and an antigen-binding fragment are well known and can be found in the prior art, such as in the Antibodies: A Laboratory Manual, Chapters 5-8 and 15, by Cold Spring Harbor. The engineered antibodies or antigen-binding fragments thereof in the present disclosure can be prepared and purified by conventional methods. For example, cDNA sequences encoding heavy and light chains may be cloned and recombined into an expression vector. The recombinant vector for immunoglobulin expression may be stably transfected into CHO cells. A stable clone is obtained by expressing antibodies that specifically bind to human antigens. The positive clone is expanded in a serum-free medium in a bioreactor to produce the antibodies. The culture medium into which the antibodies are secreted may be purified and collected by conventional techniques. The antibodies may be filtrated and concentrated using conventional methods. Soluble mixtures and multimers may also be removed by conventional methods, such as molecular sieves and ion exchange.

As used herein, the term "individual" or "subject" refers to any animal, such as a mammal or a marsupial. The individual of the present disclosure includes, but is not limited to, a human, a non-human primate (e.g., a cynomolgus monkey, a rhesus monkey or a macaque monkey of another type), a mouse, a pig, a horse, a donkey, a bovine, a sheep, a rat, and a poultry of any kind.

As used herein, the term "disease", "condition", "disorder" or the like refers to any alteration or dysregulation that impairs or interferes with the normal function of a cell, tissue or organ. For example, the "disease" includes, but is not limited to, a tumor, an infection with a pathogen, an autoimmune disease, a T-cell-dysfunctional disease, or deficiency in immunotolerance (e.g., graft rejection), and the like.

As used herein, the term "tumor" refers to a disease characterized by pathological proliferation of cells or tissues, and their subsequent migration or invasion to other tissues or organs. Tumor growth is generally uncontrolled and progressive, and does not induce or inhibit normal cell proliferation. Tumor includes "cancer", which refers generally to all malignancies.

As used herein, the term "treatment" refers to a clinical intervention in attempting to alter a person or treat a disease caused by a cell, either prophylactically or in a clinically pathological process. Therapeutic effects include, but are not limited to, prevention of the onset or recurrence of a disease, alleviation of symptoms, reduction of direct or indirect pathological consequences of any disease, prevention of metastasis, slowing of the rate of progression of a disease, amelioration or alleviation of a disease state, alleviation or improvement of prognosis, and the like.

As used herein, the term "in combination with" involves a treatment regimen that provides at least two or more than two different therapies that may be physical, such as radiation therapy, or may be chemical, such as administering to a subject a medicine that also includes medicines for combination use, to achieve a specified therapeutic effect. "Medicines for combination use" refers to a combination comprising two or more pharmaceutical formulations each having an active ingredient, which need to be used in combination when administered to a subject. The active ingredients may be mixed together to form a single administration unit, or may be separately used as individual administration units, respectively. At the time of administration, the different pharmaceutical formulations may be administered substantially synchronously, simultaneously or sequentially.

Recurrence-free survival (RFS) is defined as the period from the time of randomization to the time of first recording of disease recurrence (local, regional, or distant), or death from any cause, whichever occurs first.

Overall survival (OS) refers to the period between the initiation of study treatment and the subject's death from any cause. Patients who were still at follow-up as of the analysis cutoff date (Cut-off) were censored at the analysis cutoff date, and subjects who were lost to follow-up were censored at the date of their last contact.

Progression-free survival (PFS) is the period between the initiation of study treatment and the subject's first imaging assessment of disease progression or death of any cause (whichever occurs first).

Objective response rate (ORR) is defined as the best ORR confirmed during the study, including cases of complete response (CR) and partial response (PR). According to RECIST vl.l, when PR or CR is first present, confirmation needs to be made by additional imaging examination of lesions at ≥4 weeks.

The disease control rate (DCR) refers to the percentage of cases with an optimal efficacy evaluation of "CR+PR+SD".

Complete response (CR): all target lesions disappear and the short diameters of all pathological lymph nodes (including target and non-target nodules) must be reduced to <10mm.

The partial response (PR): the sum of the diameters of target lesions is reduced by at least 30% from the baseline level.

Disease progression (PD): a relative increase by at least 20% in the sum of the diameters of all target lesions measured throughout the course of the experimental study with reference to the minimum of the sum of the diameters of all target lesions measured (with reference to the baseline value if the baseline measurement is the minimum); and otherwise the absolute value of the sum of the diameters must be increased by at least 5 mm (the appearance of one or more new lesions is also considered to be disease progression).

Stable disease (SD): the degree of reduction of a target lesion does not reach PR, the degree of its increase does not reach a PD level, and it is between the two, where the minimum value of the sum of the diameters can be used as a reference for the study.

Duration of response (DOR) is defined as the period between the first assessment of a tumor as CR or PR (whichever is recorded first) and the first assessment as PD or death.

A treatment emergent adverse event (TEAE) refers to an adverse event occurring during the treatment.

A treatment-related adverse event (TRAE) refers to an adverse event associated with a study drug occurring during the treatment.

A serious adverse event (SAE) is an adverse medical event (AE), such as death, life-threatening, permanent or serious disability or loss of function, needs for hospitalization or prolongation of hospitalization time, and a congenital abnormality or birth defect, which occurs after a subject receives an investigational drug.

Dose-limiting toxicity (DLT) is defined as grade ≥3 non-hematologic toxicity or grade ≥ 4 hematologic toxicity occurring within 21 days (1 cycle) after the first dose.

The maximum tolerated dose (MTD) is defined as the highest dose level of the drug at which no more than 1 DLT occurred in 6 subjects during the 21-day (1 cycle) treatment period after the first dose.

### Examples

The present disclosure will be further illustrated below in connection with specific Examples. It is to be understood that these Examples are merely illustrative of the present disclosure and are not intended to limit the scope of the present disclosure.

### Example 1 Obtaining the mixture of antibodies ZPML265

The mixture of antibodies was produced by a single host cell line using the method disclosed in PCT/US2017/030676, and the mixture of antibodies contains two active ingredients, a recombinant humanized IgG1 monoclonal antibody targeting human CTLA4 and a recombinant humanized IgG4 monoclonal antibody targeting human PD1. The mixture of antibodies can specifically bind to both of human CTLA4 and PD1. Both antibodies were expressed simultaneously by the single host cell line, collected, purified, and combined with a pharmaceutically acceptable carrier to form a single pharmaceutical preparation of the mixture of antibodies, ZPML265. The amino acid sequences of each of the anti-CTLA4 antibody and the anti-PD1 antibody are shown in Table 1 below.

**Table 1 Amino acid sequence of each component of the mixture of antibodies ZPML265**

| Name | SEQ ID NO: |
|---|---|
| Heavy chain HCDR1 of anti-CTLA4 antibody | 1 |
| Heavy chain HCDR2 of anti-CTLA4 antibody | 2 |
| Heavy chain HCDR3 of anti-CTLA4 antibody | 3 |
| Light chain LCDR1 of anti-CTLA4 antibody | 4 |
| Light chain LCDR2 of anti-CTLA4 antibody | 5 |
| Light chain LCDR3 of anti-CTLA4 antibody | 6 |
| Heavy chain variable region VH of anti-CTLA4 antibody | 7 |
| Light chain variable region VL of anti-CTLA4 antibody | 8 |
| Heavy chain HCDR1 of anti-PD1 antibody | 9 |
| Heavy chain HCDR2 of anti-PD1 antibody | 10 |
| Heavy chain HCDR3 of anti-PD1 antibody | 11 |
| Light chain LCDR1 of anti-PD1 antibody | 12 |
| Light chain LCDR2 of anti-PD1 antibody | 13 |
| Light chain LCDR3 of anti-PD1 antibody | 14 |
| Heavy chain variable region VH of anti-PD1 antibody | 15 |
| Light chain variable region VL of anti-PD1 antibody | 16 |
| Heavy chain HC of anti-CTLA4 antibody | 17 |
| Light chain LC of anti-CTLA4 antibody | 18 |
| Heavy chain HC of anti-PD1 Antibody | 19 |
| Light chain LC of anti-PD1 antibody | 20 |

### Example 2 Obtaining ZPML264

ZPML264 injection contained only one active ingredient, namely the recombinant humanized IgG4 monoclonal antibody targeting human PD1 contained in ZPML265, the sequence of which is shown in Table 1. After expression of the antibody by a host cell line, the antibody was collected, purified, and combined with a pharmaceutically acceptable carrier to form a pharmaceutical preparation ZPML264.

### Example 3 A multicenter, open phase Ib study to evaluate the effectiveness and safety of ZPML265 in patients with advanced malignant solid tumors

### Dosing

A total of 419 subjects were enrolled in a phase Ib clinical study conducted in China. 25 cases with hepatocellular carcinoma were included. The patients were aged above 18 years, with American Eastern Cooperative Oncology Group (ECOG) performance status scores of 0 or 1 and expected survival time of ≥3 months. The functional levels of vital organs before the first administration of the investigational drug were as follows:
1) absolute neutrophil count ≥ 1.5× 10⁹/L;
2) platelets ≥ 75 × 10⁹/L;
3) hemoglobin ≥ 90g/L;
4) serum albumin ≥ 30g/L;
5) aspartate aminotransferase (AST) and alanine transaminase (ALT) ≤ 2.5×upper limit of normal (ULN) (for patients with liver cancer or liver metastases, ≤ 5×ULN was permissible);
6) total bilirubin ≤ 1.5×ULN (for patients with Gilbert syndrome, ≤ 3 ×ULN was permissible);
7) international normalized ratio (INR) ≤ 1.5 [for patients with liver cancer, INR ≤ 2.3 or prothrombin time (PT) prolonged by ≤ 6 seconds]; activated partial thromboplastin time (APTT) ≤ 1.5×ULN;
8) serum creatinine ≤ 1.5×ULN; if the creatinine level of a subject was > 1.5×ULN, creatinine clearance (CrCl) (calculated according to the Cockcroft-Gault formula) was required to be ≥ 50mL/min; and
9) left ventricular ejection fraction (LVEF) > 50%.

Dosing Regimen: ZPML265 being administered at a dose of 5 mg/kg once every three weeks (Q3W) by intravenous infusion.

### Results for effectiveness

A total of 25 subjects with advanced hepatocellular carcinoma were enrolled in this study to receive treatment with a dose of 5 mg/kg. None of the 25 subjects with hepatocellular carcinoma achieved CR or PR, with 10 (40.0%) subjects having an optimal efficacy assessment outcome of SD, a confirmed ORR of 0% (95% CI: 0.000, 13.719), and a confirmed DCR of 40.0% (95% CI: 21.125, 61.335). Of the subjects with hepatocellular carcinoma, 3 subjects had not been subjected to a systemic therapy, of which 1 subject had an optimal efficacy assessment outcome of SD and 2 subjects had PD. Of the subjects who had been subjected to a systemic therapy, 5 cases had not been subjected to an immunotherapy, and 3 cases had an optimal efficacy assessment of SD, with a confirmed DCR of 60.0% (95% CI: 14.663, 94.726); 17 cases had been subjected to an immunotherapy, and 6 cases had an optimal efficacy assessment of SD, with a confirmed DCR of 35.3% (95% CI: 14.210, 61.672). It should be noted that of the subjects that had been subjected to an immunotherapy, 2 subjects were found to have a first efficacy assessment of PD based on the RECISTvl.l criteria, but continued to receive the study therapy, with tumor shrinkage reaching PR and sustained maintenance of PR status.

Summary of efficacy: ZPML265 as a monotherapy showed certain efficacy on hepatocellular carcinoma.

### Results for safety

Safety data aggregated data from 609 subjects treated with ZPML265. As of May 31, 2022, 546 (89.7%) patients experienced TEAEs and 451 (74.1%) patients experienced TEAEs associated with ZPML265. Grade ≥3 TEAEs and SAEs were reported in 187 (30.7%) and 156 (25.6%) patients, respectively. Grade ≥3 TEAEs associated with ZPML265 and SAEs associated with ZPML265 were reported in 102 (16.7%) and 75 (12.3%) patients, respectively.

In contrast to ZPML265 monotherapy, another Meta-analysis of 2664 patients with nivolumab in combination with ipilimumab showed that the overall incidence of treatment-related serious adverse events was 29.6%, and the incidence of grade ≥3 TRAEs was 39.9%, which was higher than the data associated with ZPML265 monotherapy.

From the available data, it was known that the treatment safety was acceptable and no new safety signal was found compared to other immunotherapy drugs.

### Example 4 A phase Ib/II clinical study to evaluate the safety, pharmacokinetics and preliminary efficacy of ZPML265 or ZPML264 injection in combination with bevacizumab injection in patients with advanced liver cancer

ZPML265 or ZPML264 injection was used in combination with bevacizumab injection in an open, multicenter, phase Ib/II clinical study of patients with unresectable or metastatic hepatocellular carcinoma that had not been subjected to a systemic therapy, which were divided into three cohorts, cohort A, cohort B and cohort C, with 50 subjects enrolled in cohort A, 26 subjects enrolled in cohort B, and 40 subjects enrolled in cohort C. The patients were aged above 18 years, with American Eastern Cooperative Oncology Group (ECOG) performance status scores of 0 or 1 and expected survival time of ≥3 months. The functional levels of vital organs before the first administration of the investigational drug were as follows:
1) absolute neutrophil count ≥ 1.5 × 10⁹/L;
2) platelets ≥ 75 × 10⁹/L;
3) hemoglobin ≥ 90g/L;
4) serum albumin ≥ 30g/L;
5) AST and ALT ≤ 5 ×upper limit of normal (ULN);
6) total bilirubin ≤ 1.5×ULN (for patients with Gilbert syndrome, ≤ 3 ×ULN was permissible);
7) serum creatinine ≤ 1.5×ULN; if the creatinine level of a subject was > 1.5×ULN, creatinine clearance (CLcr) calculated by the Cockcroft-Gault formula was ≥ 50mL/min;
8) left ventricular ejection fraction (LVEF) > 50%;
9) proteinuria < 2+ (where urinary protein was ≥ 2+, 24h urinary protein quantification should be performed, and when it was ≤ 1g, the subject could be included);
10) for patients that had not received an anticoagulant therapy, international normalized ratio (INR) or activated partial thromboplastin time (APTT) ≤ 2×ULN.

### Dosing Regimen

Cohort A: ZPML265 being administered at a dose of 5 mg/kg, Q3W, by intravenous infusion; and bevacizumab injection being administered at a dose of 7.5mg/kg or 15 mg/kg, Q3W, by intravenous infusion.

Cohort B: ZPML264 being administered at a fixed dose of 200 mg, Q3W, by intravenous infusion; and bevacizumab injection being administered at a dose of 7.5mg/kg or 15 mg/kg, Q3W, by intravenous infusion.

Cohort C: ZPML265 being administered at a dose of 7.5mg/kg, Q3W, by intravenous infusion, and bevacizumab injection being administered at a dose of 7.5mg/kg or 15 mg/kg, Q3W, by intravenous infusion.

### Results for safety

As of the cut-off point for data analysis of 20 February, 2023, cohort A safety analysis set included 50 subjects; and cohort C safety analysis set included 40 subjects. This study was in progress, and the preliminary results for safety in cohort A and cohort C were summarized as follows:

### Cohort A

Of the 50 subjects in cohort A, a total of 49 (98.0%) subjects experienced TEAEs, and 43 cases (86.0%) experienced TRAEs, of which 42 cases (84.0%) experienced TEAEs associated with ZPML265, 40 cases (80.0%) experienced TEAEs associated with bevacizumab; 16 cases (32.0%) experienced grade ≥3 TEAEs associated with ZPML265, 14 cases (28.0%) experienced grade ≥3 TEAEs associated with bevacizumab; 6 cases (12.0%) experienced SAEs associated with ZPML265, and 6 cases (12.0%) experienced SAEs associated with bevacizumab. 26 cases (52.0%) experienced immune-related TEAEs, and 7 cases (14.0%) experienced grade ≥3 immune-related TEAEs. 18 cases (36.0%) experienced TEAEs associated with ZPML265 leading to drug discontinuation, and 1 case (2.0%) experienced permanent drug discontinuation associated with ZPML265. 1 case (2.0%) experienced adverse events associated with ZPML265 leading to withdrawal from the trial, and no adverse event associated with ZPML265 leading to death was observed.

### Cohort C

Of the 40 subjects in cohort C (ZPML265 7.5mg/kg+ bevacizumab 15 mg/kg), a total of 40 cases (100.0%) experienced TEAEs, and 35 cases (87.5%) experienced TRAEs, of which 32 cases (80.0%) experienced TEAEs associated with ZPML265, 29 cases (72.5%) experienced TEAEs associated with bevacizumab; 6 cases (15.0%) experienced grade ≥3 TEAEs associated with ZPML265, 6 cases (15.0%) experienced grade ≥3 TEAEs associated with bevacizumab; 3 cases (7.5%) experienced SAEs associated with ZPML265, and 2 cases (5.0%) experienced SAEs associated with bevacizumab. 11 cases (27.5%) experienced immune-related TEAEs. No grade ≥3 immune-related TEAEs were observed. 9 cases (22.5%) experienced TEAEs associated with ZPML265 leading to drug discontinuation. No permanent drug discontinuation associated with ZPML265 was observed. No adverse events associated with ZPML265 leading to withdrawal from the trial and no adverse events associated with ZPML265 leading to death were observed.

The above data showed that both of 5mg/kg and 7.5mg/kg of ZPML265 in combination with bevacizumab had good safety, and no significant increase in toxicity was observed as compared to the PD-1 monoclonal antibody in combination with bevacizumab.

### Results for effectiveness

As of February 20, 2023, a total of 108 subjects were included in the efficacy evaluable set, including 47 cases in cohort A, 26 cases in cohort B, and 35 cases in cohort C.

In cohort A, the overall objective response rate (ORR) was 38.3% (95% CI: 24.5%, 53.6%), with 1 (2.1%) subject achieving CR and 17 (36.2%) subjects having an optimal efficacy assessment outcome of PR. 17 (36.2%) subjects had an optimal efficacy assessment outcome of SD. Therefore, the DCR was 74.5% (95% CI: 59.7%, 86.1%). 12 (25.5%) subjects had an optimal efficacy assessment outcome of PD. The median progression-free survival (mPFS) was 7.0 (95% CI, 3.1-9.9) months.

In cohort B, the overall ORR was 23.1% (95% CI: 9.0%, 43.6%), with 1 (3.8%) subject achieving CR and 5 (19.2%) subjects having an optimal efficacy assessment outcome of PR. 12 (46.2%) subjects had an optimal efficacy assessment outcome of SD. Therefore, the DCR was 69.2% (95% CI: 48.2%, 85.7%). 7 (26.9%) subjects had an optimal efficacy assessment outcome of PD. The mPFS was 5.4 (95% CI 2.4-8.5) months.

In cohort C, the overall ORR was 34.3% (95% CI: 19.1%, 52.2%), with no subject achieving CR, and 12 subjects (34.3%) having an optimal efficacy assessment outcome of PR. 17 (48.6%) subjects had an optimal efficacy assessment outcome of SD. Therefore, the DCR was 82.9% (95% CI: 66.4%, 93.4%). 6 (17.1%) subjects had an optimal efficacy assessment outcome of PD.

As of the cut-off date for data analysis, ZPML265 5mg/kg+ bevacizumab was comparable to ZPML265 7.5mg/kg+ bevacizumab, and both showed superior efficacy over ZPML264 monoclonal antibody + bevacizumab. The ZPML265 7.5mg/kg+ bevacizumab group had a short follow-up time, and its ORR data were not yet fully established, but with an upward trend.

### Example 5 A randomized, controlled, open, multicenter phase II/III clinical study to evaluate combination medication of ZPML265 versus sintilimab in combination with bevacizumab in first-line treatment of patients with advanced hepatocellular carcinoma

In a phase II/III clinical study conducted in China, approximately 668 patients with unresectable or metastatic hepatocellular carcinoma that had not been subjected to a systemic therapy were scheduled to be enrolled. The patients were required to be aged from 18 years to 75 years, at stage C or at stage B which was not suitable for radical surgery and/or topical treatment according to the Barcelona Clinical HCC staging (BCLC staging), with American Eastern Cooperative Oncology Group (ECOG) performance status scores of 0 or 1, and with grade A or grade B of ≤7 according to the Child-Pugh liver function grading. The functional levels of vital organs before the first administration of the investigational drug were as follows:
1) absolute neutrophil count ≥ 1.5× 10⁹/L;
2) platelets ≥ 75 × 10⁹/L;
3) hemoglobin ≥ 90g/L;
4) albumin ≥ 29g/L;
5) each of alanine aminotransferase (ALT), aspartate aminotransferase (AST) and alkaline phosphatase (AKP) ≤ 5×ULN;
6) total bilirubin (TBIL) ≤ 2×ULN;
7) creatinine clearance (CrCl) calculated by the Cockcroft-Gault formula ≥ 50mL/min;
8) international normalized ratio (INR) ≤ 2, or prothrombin time (PT) exceeding the upper limit of the normal range by ≤ 6 seconds; and
9) proteinuria < 2+ (where urinary protein was ≥ 2+, 24h urinary protein quantification should be performed, and when the amount of 24h urinary protein was < 1.0g, inclusion could be made).

### Dosing Regimen

### Phase II clinical study

Test group 1 received ZPML265 in combination with bevacizumab and chemotherapy in the following administration order. ZPML265 was administered at a dose of 7.5mg/kg on D1 via intravenous drip, Q3W. Bevacizumab was administered at a dose of 15 mg/kg on D1 via intravenous drip, Q3W. Chemotherapy was the XELOX regimen, in which oxaliplatin was administered at a dose of 85mg/m² on D1 via intravenous drip, Q3W; and capecitabine was administered at a dose of 1000mg/m² orally, twice a day, from D1-14, and then with a rest for 7 days, Q3W.

Test group 2 received ZPML265 in combination with bevacizumab in the following administration order. ZPML265 was administered at a dose of 7.5mg/kg on D1 via intravenous drip, Q3W. Bevacizumab was administered at a dose of 15 mg/kg on D1 via intravenous drip, Q3W.

Test group 3 received ZPML265 in combination with chemotherapy in the following administration order. ZPML265 was administered at a dose of 7.5mg/kg on D1 via intravenous drip, Q3W. Chemotherapy was the XELOX regimen, in which oxaliplatin was administered at a dose of 85mg/m² on D1 via intravenous drip, Q3W; and capecitabine was administered at a dose of 1000mg/m² orally, twice a day, from D1-14, and then with a rest for 7 days, Q3W.

The control group received treatment with sintilimab in combination with bevacizumab in the following administration order. Sintilimab was administrated at a dose of 200 mg on D1 via intravenous drip, Q3W; and bevacizumab was administrated at a dose of 15 mg/kg on D1 via intravenous drip, Q3W.

### Phase III clinical study

Dosing regimens for selected test groups and control group were identical to those in the phase II clinical study.

## Claims

1. A pharmaceutical composition for treating hepatocellular carcinoma, comprising an effective amount of a mixture of antibodies of anti-CTLA4 and anti-PD1, the mixture of antibodies being produced by a single host cell comprising both a nucleic acid encoding an anti-CTLA4 antibody and a nucleic acid encoding an anti-PD1 antibody, wherein the sequences of heavy chain HCDR1, HCDR2 and HCDR3 of the anti-CTLA4 antibody are set forth in SEQ ID NOs:1, 2 and 3, respectively; the sequences of light chain LCDR1, LCDR2 and LCDR3 of the anti-CTLA4 antibody are set forth in SEQ ID NOs:4, 5 and 6, respectively; the sequences of heavy chain HCDR1, HCDR2 and HCDR3 of the anti-PD1 antibody are set forth in SEQ ID NOs:9, 10 and 11, respectively; and the sequences of light chain LCDR1, LCDR2 and LCDR3 of the anti-PD1 antibody are set forth in SEQ ID NOs: 12, 13 and 14, respectively.

2. The pharmaceutical composition of claim 1, wherein the sequence of heavy chain variable region of the anti-CTLA4 antibody is set forth in SEQ ID NO:7, the sequence of light chain variable region of the anti-CTLA4 antibody is set forth in SEQ ID NO:8, the sequence of heavy chain variable region of the anti-PD1 antibody is set forth in SEQ ID NO: 15, and the sequence of light chain variable region of the anti-PD1 antibody is set forth in SEQ ID NO:16.

3. The pharmaceutical composition of claim 1, wherein the sequence of heavy chain of the anti-CTLA4 antibody is set forth in SEQ ID NO: 17, the sequence of light chain of the anti-CTLA4 antibody is set forth in SEQ ID NO:18, the sequence of heavy chain of the anti-PD1 antibody is set forth in SEQ ID NO: 19, and the sequence of light chain of the anti-PD1 antibody is set forth in SEQ ID NO:20.

4. The pharmaceutical composition of any one of claims 1-3, wherein the hepatocellular carcinoma is unresectable or metastatic hepatocellular carcinoma that has not been subjected to a systemic therapy.

5. The pharmaceutical composition of claim 4, wherein the mixture of antibodies is administered at a dose of 5 mg/kg or 7.5mg/kg once every three weeks by intravenous infusion on day 1, with one treatment cycle of 21 days.

6. The pharmaceutical composition of claim 4, further comprising a chemotherapeutic drug and/or an anti-angiogenic agent.

7. The pharmaceutical composition of claim 6, wherein the chemotherapeutic drug is oxaliplatin and capecitabine, and/or wherein the anti-angiogenic agent is bevacizumab.

8. The pharmaceutical composition of claim 7, wherein bevacizumab is administered at a dose of 7.5mg/kg or 15 mg/kg by intravenous infusion on day 1 of each dosing cycle, with one dosing cycle of three weeks.

9. The pharmaceutical composition of claim 7, wherein oxaliplatin is administered at a dose of 85mg/m² by intravenous infusion on day 1 of each dosing cycle, with one dosing cycle of three weeks; and capecitabine is administered at a dose of 1000mg/m² orally from day 1 to day 14 of each cycle, with one dosing cycle of three weeks.

10. The pharmaceutical composition of any one of claims 1-3, wherein the hepatocellular carcinoma is hepatocellular carcinoma with high-risk recurrence factors after surgical resection or local ablation treatment.

11. The pharmaceutical composition of claim 10, wherein the mixture of antibodies is administered at a dose of 5 mg/kg or 7.5mg/kg by intravenous infusion on day 1 of each dosing cycle, with one dosing cycle of three weeks.

12. A method for treating hepatocellular carcinoma comprising administering to a subject the pharmaceutical composition of any one of claims 1-11.

13. Use of a mixture of antibodies comprising an effective amount of anti-CTLA4 and anti-PD1 in the manufacture of a pharmaceutical composition for treating hepatocellular carcinoma, the mixture of antibodies being produced by a single host cell comprising both a nucleic acid encoding an anti-CTLA4 antibody and a nucleic acid encoding an anti-PD1 antibody, wherein the sequence of heavy chain of the anti-CTLA4 antibody is set forth in SEQ ID NO: 17, the sequence of light chain of the anti-CTLA4 antibody is set forth in SEQ ID NO: 18, the sequence of heavy chain of the anti-PD1 antibody is set forth in SEQ ID NO:19, and the sequence of light chain of the anti-PD1 antibody is set forth in SEQ ID NO:20; the hepatocellular carcinoma is unresectable or metastatic hepatocellular carcinoma that has not been subjected to a systemic therapy; or the hepatocellular carcinoma is hepatocellular carcinoma with high-risk recurrence factors after surgical resection or local ablation treatment.

14. The use of claim 13, wherein the pharmaceutical composition further comprises a chemotherapeutic drug and/or an anti-angiogenic agent.

15. The use of claim 14, wherein the chemotherapeutic agent is oxaliplatin and capecitabine, and/or wherein the anti-angiogenic agent is bevacizumab.
